# EUROPÄISCHE PATENTSCHRIFT

(11) **EP 0 487 771 B1**
(45) Veröffentlichungstag und Bekanntmachung des Hinweises auf die Patenterteilung: **15.06.1994**
(21) Anmeldenummer: 90122736.3
(22) Anmeldetag: 28.11.1990
(51) Int. Cl.: D06P 1/607, D06P 3/24, D06P 5/12

(54) **Verwendung von Dialkylmonoaminalkoxylat-Verbindungen zum Reservieren von anionischen Färbungen auf Polyamidfasern**
Use of alkoxylated dialkylmonoamine compounds for the reserve of anionic dyeings of polyamide-fibrous material
Emploi de dialkylmonoamines alcoxylées pour réserve de teintures anioniques de produits textiles polyamides

(43) Veröffentlichungstag der Anmeldung: 03.06.1992
(73) Patentinhaber: ZSCHIMMER & SCHWARZ GmbH & Co. CHEMISCHE FABRIKEN, D-56108 Lahnstein (DE)
(72) Erfinder: Pospischil, Karl-Heinz, Dr., W-5423 Braubach (DE); Bung, Hans-Peter, W-5419 Ötzingen (DE)

(56) Entgegenhaltungen:
- DE-A- 3 623 967
- FR-A- 1 505 749
- GB-A- 835 267

## Beschreibung

Die Erfindung betrifft die Verwendung von Dialkylmonoaminalkoxylat-Verbindungen zum Erzeugen von Reservierungs-, Farbmusterungs- und Multicoloreffekten bei Färbungen mit anionischen Farbstoffen auf Polyamid-Fasermaterial.

Es ist bekannt, daß mit alkoxylierten Monoalkylaminverbindungen gute Reservierungseffekte und Buntreserven gegenüber anionischen Farbstoffen erzielbar sind. So beschreibt die DE-A-32 22 516 ein Verfahren zur Herstellung von Reserven auf mit anionischen Farbstoffen anfärbbaren textilen Materialien, bei dem ein alkoxyliertes Monoalkylamin, Monoalkylpolyamin oder Fettsäureamid, das gegenüber anionischen Farbstoffen Affinität besitzt und reservierend wirkt, in Form der Base, als Salz oder quaterniert lokal appliziert und danach mit einer sauren Färbeflotte, die mindestens einen anionischen Farbstoff mit einem K'-Wert ≧ 5 enthält, überfärbt wird. Der K'-Wert ist eine färbereitechnische Kennzahl für das Färbeverhalten anionischer Farbstoffe hinsichtlich ihrer Kombinationsmöglichkeiten; Näheres ist z. B. in Bayer-Farbenrevue 21 (1972) und Melliand Textilberichte 1973, 641 ff. beschrieben. Je nach Art des Substrates und der Farbstoffe wird der pH-Wert der Flotte oder Paste vorzugsweise auf 7 - 10 eingestellt.

Die DE-A-30 03 192 beschreibt ein Reservierungsverfahren für anionische Färbungen auf Polyamidfasern, bei welchem zunächst eine Fondfärbung mit einem anionischen Farbstoff durchgeführt und dann lokal mit einer Flotte oder Paste übermustert wird, welche ein alkoxyliertes Monoalkylamin, Monoalkylpolyamin oder Fettsäureamid der gleichen Art wie oben als Reservierungsmittel enthält.

Auch in der DE-A-36 23 967 wird die Herstellung von Reserve- oder Bunteffekten auf Polyamidtextilien beschrieben, wobei zunächst mit einer anionische Farbstoffe enthaltenden Färbeflotte bei pH 4 - 7 behandelt und danach eine Flotte oder Paste appliziert wird, die als reservierende Verbindung eine 5-10-fach alkoxylierte Monoalkyldiamin-Verbindung enthält.

Es wurde nun überraschenderweise gefunden, daß mit Dialkylmonoaminalkoxylat-Verbindungen in mindestens teilweise quaternierter Form wesentlich bessere Farbmusterungen, Weiß- und Buntreservierungseffekte als nach den bekannten Verfahren erhalten werden.

Hierzu werden Dialkylmonoaminalkoxylat-Verbindungen der Formel 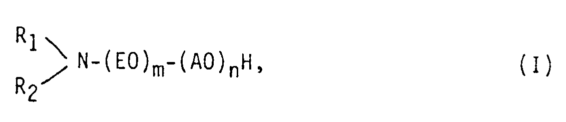
worin
- R₁ und R₂: Fettalkylreste mit C₈-C₂₂, gleich oder verschieden, gesättigt oder ungesättigt,
- E: Ethylen-Rest,
- A: Ethylen- oder Propylen-Rest,
- m: 1 bis 2,
- n: 1 bis 50
bedeuten, in mindestens teilweise quaternierter Form in farbstofffreien oder farbstoffhaltigen Reservierungspasten zum Herstellen von Farbmusterungen auf synthetischem Polyamidfasermaterial unter oder auf Färbungen mit anionischen Farbstoffen verwendet.

Das Textilmaterial wird vor oder nach dem lokalen Aufbringen der farbstoffhaltigen bzw. farbstofffreien Reservierungspasten mit einer anionische Farbstoffe enthaltenden Färbeflotte behandelt und dann einer fixierenden Dämpfbehandlung unterworfen, wobei die in der Fondfärbung eingesetzten Farbstoffe K'-Werte ≧ 5, die in den Reservierungspasten verwendeten Farbstoffe K'-Werte ≦ 5 haben und die Differenz dieser K'-Werte mindestens 2 beträgt. Die erfindungsgemäßen, als Reservierungsmittel verwendeten alkoxylierten Dialkylmonoamin-Verbindungen liegen in mindestens teilweise quaternierter Form vor und wirken sowohl im sauren als auch im alkalischen Bereich gegenüber anionischen Farbstoffen reservierend. Insbesondere ist dies in dem technologisch vorteilhaften sauren pH-Bereich von 3 bis 7 der Fall, darüber hinaus ist aber auch im alkalischen pH-Bereich von 7 bis 11 gute reservierende Wirkung vorhanden. Dieser anwendbare Gesamtbereich von pH 3 bis 11 der Reservierungspasten bietet in der Praxis den Vorteil eines wesentlich sichereren Arbeitens, und es werden klarere Überdrucke und Buntreserven mit optimaler Farbausbeute erzielt.

In den durch die allgemeine Formel beschriebenen Dialkylmonoaminalkoxylaten sind R₁ und R₂, die gleich oder verschieden sein können, längerkettige gesättigte oder ungesättigte, geradkettige oder verzweigte Alkyl-Reste mit 8 bis 22 Kohlenstoffatomen, wie z. B. Octyl, Nonyl, Decyl, Undecyl, Dodecyl, Tridecyl, Tetradecyl, Pentadecyl, Hexadecyl, Stearyl, Behenyl, Oleyl sowie die entsprechenden, in nativen oder technischen Fetten und Ölen wie z. B. in Talgölen, Sojaölen, Kokosölen und Kokosfetten enthaltenen Alkyl- und Alkenylreste. Zur Quaternierung kommen übliche Mittel wie z.B. Benzylchlorid, Methylchlorid, Dimethylsulfat, Diethylsulfat in Frage.

Ein Beispiel für die beschriebenen Reservierungsmittel stellt die folgende Verbindung dar, in der die Quaternierung mit Benzylchlorid erfolgte: 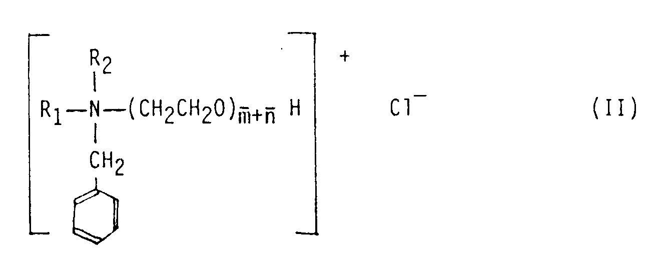
mit
- R₁ und R₂: = C_{16,18}H_{33,37}
- m̅ + n̅: = mittlerer Ethoxylierungsgrad 30

Mit solchen Verbindungen können bei Färbungen anionischer Farbstoffe mit passenden K'-Werten auf Polyamidfasermaterialien, insbesondere auch Polyamid-Teppichmaterialien, Reservierungs-, Farbmusterungseffekte wie Bi- und Multicoloreffekte, Hell-Dunkel-Effekte, Kontrastfarb-Effekte usw. erzielt werden.

Die Anwendung der quaternierten alkoxylierten Dialkylmonoamin-Verbindungen erfolgt in Form von verdickten Flotten oder Pasten. Sie werden entweder auf einen vorgefärbten Fond nachträglich lokal appliziert oder auf die ungefärbte Ware zunächst lokal aufgebracht und danach durch eine Fondfärbung überfärbt. Die Reihenfolge dieser Schritte kann variiert werden. Bei der Applikation können bekannte Techniken wie Rotations- und Schablonendruckverfahren, Begießen, Bespritzen, Betropfen, Imprägnieren usw. angewendet werden, wobei der gesamte Prozeß in einem Arbeitsgang durchführbar ist

Als Farbstoffe, die für die Fondfärbung vor oder nach Applizierung der Reservierungsmittel eingesetzt werden, eignen sich anionische Farbstoffe, insbesondere 1:2- oder 1:1-Metallkomplexfarbstoffe, die eine oder mehrere wasserlöslich machende Gruppen, vornehmlich Sulfogruppen, enthalten. Geeignete Farbstoffe müssen unabhängig von der Reihenfolge der Operationen einen K'-Wert ≧ 5 besitzen.

Enthält die Reservierungspaste ebenfalls anionische Farbstoffe, so müssen diese K' ≦ 5 haben, und die Differenz zwischen den K'-Werten der in der Fondfärbung und der in den Reservierungspasten befindlichen Farbstoffe muß mindestens 2 betragen. Es können auch mehrere verschiedene Reserven aufgebracht werden, die verschiedene Farbstoffe oder deren Kombinationen enthalten können, so daß Multicoloreffekte entstehen. Die K'-Werte dieser Farbstoffe müssen kleiner als 5 sein und um mindestens zwei Stufen niedriger liegen als bei den Farbstoffen der Färbeflotte.

Die Applikation von Fondfärbung und Reservierungspaste kann in beliebiger Reihenfolge Naß-auf-Naß direkt aufeinander erfolgen, ohne daß Zwischenschritte wie Zwischentrocknung oder Zwischendämpfen erforderlich sind. Anschließend werden die in Fond und lokalen Reserven aufgebrachten Farbstoffe durch Dämpfen gemeinsam fixiert; danach wird ausgewaschen und gespült. Durch die Gegenwart der reservierenden Verbindungen werden Farbstoffe mit K' ≧ 5 an der Fixierung gehindert, während Farbstoffe mit K' ≦ 5 in ihrer Fixierung nicht behindert werden. Je größer die Differenz zwischen den K'-Werten der in der Fondfärbung und der in der Reservierungspaste vorhandenen Farbstoffe, um so bessere Kontraste werden erzielt.

Die reservierende Verbindung wird in einer Menge von 1 - 100 Gewichtsteilen, bezogen auf 1000 Gewichtsteile Reservierungspaste, eingesetzt; vorzugsweise wird in dem Bereich von 2 - 30 Gewichtsteilen gearbeitet.

Flotten und Pasten können neben den reservierenden Verbindungen und den anionischen Farbstoffen weitere übliche Zusatzmittel wie Verdickungsmittel, Entschäumungsmittel, Antifrostingmittel, Salze, optische Aufheller usw. enthalten. Die Viskosität der Reservierungspaste muß höher liegen als diejenige der Fondfärbeflotte. Der pH-Wert der Reservierungspaste bzw. -flotte wird in einem Bereich zwischen ca. 3 und 11 gehalten; vorzugsweise wird auf pH 5 - 6 eingestellt.

Als zu färbendes und zu reservierendes Substrat eignen sich aus synthetischen oder natürlichen Polyamidfasern hergestellte Textilien wie z. B. Polyamidteppichware.

Die Herstellung der erfindungsgemäß zu verwendenden Dialkylmonoaminalkoxylat-Verbindungen stößt jedoch auf Schwierigkeiten, die darin begründet sind, daß die beiden Alkylsubstituenten des Amins langkettig sind. Das führt beim Ethoxylieren bzw. Alkoxylieren vermutlich zu sterischen Behinderungseffekten und hat zur Folge, daß sich zweifach langkettige Dialkylamine nicht ohne weiteres alkoxylieren lassen.

Bei der bekannten Ethoxylierung von Verbindungen mit aktivem Wasserstoff-Atom, wie Alkoholen, Carbonsäuren, Aminen, Phenolen, Thiolen usw. verläuft die Reaktion mit Ethylenoxid wie vorgesehen mit oder ohne Gegenwart von Katalysator. Bei der Ethoxylierung von Alkylaminen wird dabei zunächst ohne Katalysator wenig Ethylenoxid - zwei Moleküle bei primären Aminen, ein Molekül bei sekundären Aminen - angelagert, bevor man einen Katalysator wie z. B. Kaliumhydroxid zusetzt. Die Anlagerung an primäre Fettalkylamine und an sekundäre Fettalkylamine mit einem kurzkettigen und einem langkettigen Alkylsubstituenten, wie Alkylmethylamine und Alkylethylamine, verläuft dabei glatt.

Dagegen gelingt die Ethoxylierung von Dialkylaminen mit zwei längeren Alkylketten nur sehr schwer, da das Ethylenoxid hier unter normalen Bedingungen eher mit Spuren von Verunreinigungen wie z.B. Wasser, die stets vorhanden sind, oder in Gegenwart von Katalysatoren eher mit diesen als mit dem Amin reagiert. Man erhält daher in der Regel zweiphasige Gemische, in denen die Oberphase überwiegend aus nicht oder nur zu geringen Anteilen reagiertem Dialkylamin, die Unterphase aber aus Polyglykol mit geringen aminischen Verunreinigungen besteht.

Diese Schwierigkeiten standen der Herstellung der erfindunggemäß zum Reservieren von anionischen Farbstoffen auf Polyamidfaserstoffen zu verwendenden Dialkylmonoaminalkoxylat-Verbindungen mit zwei längeren Alkylketten im Wege. Es stellte sich daher die Aufgabe, ein Verfahren zur technisch ergiebigen Herstellung dieser Alkoxylat-Verbindungen von zweifach langkettigen substituierten Aminen zu finden.

Es wurde nun gefunden, daß sich diese langkettigen Dialkylamine ohne Auftreten von Reaktionen, die nicht zu den gewünschten Alkoxylaten führen, ohne Schwierigkeiten alkoxylieren lassen, wenn man hierfür ein zweistufiges Verfahren unter modifizierten Reaktionsbedingungen anwendet.

Dieses Verfahren zur Herstellung von Dialkylmonoaminalkoxylat-Verbindungen der Formel 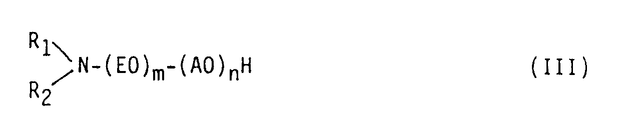
worin
- R₁ und R₂: Fettalkylreste mit C₈-C₂₂, gleich oder verschieden, gesättigt oder ungesättigt,
- E: Ethylenrest,
- A: Ethylen- oder Propylenrest,
- m: 1 bis 2,
- n: 1 bis 50
bedeuten, ist dadurch gekennzeichnet, daß
ein Dialkylmonoamin der Formel R₁-NH-R₂
zunächst in einer ersten Reaktionsstufe bei höherer Temperatur und Druck ohne Katalysator mit 1 bis 2 Mol Ethylenoxid zu einem Primäraddukt der Formel 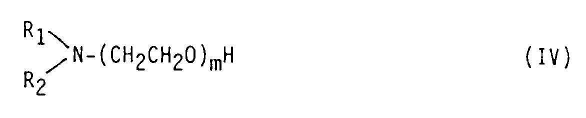
ethoxyliert wird und das entstandene Dialkylalkanolamin anschließend in einer zweiten Reaktionsstufe mit Ethylenoxid und/oder Propylenoxid in an sich bekannter Weise weiter alkoxyliert wird.

Die erhaltene Dialkylmonoaminalkoxylat-Verbindung wird dann in üblicher Weise quaterniert, wofür die bekannten Quaternierungsmittel wie z. B. Benzylchlorid, Methylchlorid, Dimethylsulfat, Diethylsulfat verwendet werden können.

Die Alkylreste R₁ und R₂ können gleich oder verschieden, gesättigt oder ungesättigt, linear oder verzweigt sein und haben Kettenlängen von 8 bis 22 Kohlenstoffatomen, wie z. B. Octyl, Nonyl, Decyl, Undecyl, Dodecyl, Tridecyl, Tetradecyl, Pentadecyl, Hexadecyl, Stearyl, Behenyl, Oleyl sowie die entsprechenden, in nativen oder technischen Fetten und Ölen wie z. B. Talgölen, Sojaölen, Kokosölen und Kokosfetten enthaltenen Alkyl- und Alkenylreste.

Durch die Anwendung von höherer Temperatur und höherem Druck in einer ersten Reaktionsstufe wird die sterische Behinderung durch die beiden langkettigen Alkylsubstituenten des Amins überwunden, wenn zunächst mit einer begrenzten, nur für die Anlagerung von ein bis höchstens zwei Molekülen ausreichenden Menge Ethylenoxid gearbeitet wird. Dies geschieht bei Temperaturen oberhalb 160 °C, vorzugsweise in einem Bereich von 180 bis ca. 200 °C, und bei einem Druck, der oberhalb 3 bar liegt, und zwar ohne Gegenwart eines Katalysators. Diese Reaktionsbedingungen müssen über mehrere Stunden, im allgemeinen etwa 4 bis 10 Stunden, aufrechterhalten werden. Unter diesen Bedingungen entsteht ein Primäraddukt der Formel IV. Dieses Dialkylalkanolmonoamin besitzt eine primäre Hydroxygruppe, die bei einer weiteren Anlagerung von Alkylenoxiden in üblicher Weise als primärer Alkohol reagieren kann. Nun wird in einer zweiten Reaktionsstufe mit Ethylenoxid und bzw. oder Propylenoxid in an sich bekannter Weise bis zum gewünschten Alkoxylierungsgrad umgesetzt. Dabei kann durch Zugabe von Katalysator in der üblichen Weise aktiviert werden.

Als Endprodukt wird eine Dialkylmonoaminalkoxylat-Verbindung erhalten, in der die Adduktkette bis zu 50 und mehr Ethylenglykol- und/oder Propylenglykol-Einheiten umfaßt. Nach diesem Verfahren lassen sich die gewünschten Alkoxylate in hoher und reproduzierbarer Ausbeute erhalten. Sie werden mit den bekannten Mitteln teilweise oder vollständig quaterniert. Die Quaternierungsprodukte eignen sich hervorragend für die Verwendung als Reservierungsmittel für Farbmusterungen auf synthetischem Polyamidfaser-Material unter oder auf Färbungen mit anionischen Farbstoffen.

### Verwendungsbeispiele

### Beispiel 1

Eine Polyamid-6,6-Velourteppichware wird nacheinander Naß-in-Naß mit Klotzflotte und Reservierungspaste behandelt. Die Klotzflotte enthält auf 1000 Gewichtsteile 10 Teile C.I. Acid Blue 232, 1 Teil Natriumacetat, 2 Teile Antifrostingmittel und 2 Teile eines alkoholhaltigen Entschäumers; der pH-Wert dieser Flotte wird auf 5,5 eingestellt. Die farbstofffreie Reservierungspaste enthält auf 1000 Gewichtsteile 20 Teile benzyliertes Dialkylaminethoxylat der Formel II, 10-20 Teile eines handelsüblichen Verdickers und 1 Teil Ammonsulfat; der pH-Wert dieser Paste wird auf 9,5 eingestellt. Die vorgewaschene Teppichware wird zunächst mit der Klotzflotte foulardiert (Flottenaufnahme 80 - 100 %, bezogen auf das Rohwarengewicht), ohne Zwischentrocknung und Dämpfprozess mit der Druckpaste bedruckt, anschließend 10 Minuten bei 100 °C im Sattdampf fixiert und nach Beendigung des Dämpfprozesses ausgewaschen. Man erhält einen dunkelblauen Teppich mit scharfrandiger, rein weißer Musterung.

### Beispiel 2

Eine Polyamid-6-Velourteppichware wird zunächst mit einer farbstofffreien Reservierungspaste bedruckt, die auf 1000 Gewichtsteile 10 Teile eines benzylierten Dialkylaminethoxylates gemäß Formel II, 20-25 Teile eines handelsüblichen Verdickers und 1 Teil Ammonsulfat enthält; der pH-Wert der Druckpaste wird auf 7,0 eingestellt. Anschließend übergießt man diese vorgedruckte Teppichware mit einer Flotte, die auf 1000 Gewichtsteile 10 Teile C.I. Acid Blue 232, 1 Teil Natriumacetat, 2 Teile eines Antifrostingmittels und 2 Teile eines alkoholischen Entschäumers enthält; der pH-Wert dieser Flotte ist auf 5,5 eingestellt. Die Flottenaufnahme beim Übergießen beträgt 300-400 %, bezogen auf das Rohwarengewicht. Anschließend läßt man 10 Minuten im Sattdampf (100-105 °C) verweilen und spült gut aus. Man erhält einen blauen Teppich mit scharf konturierter, weißer Musterung.

### Beispiel 3

Eine Polyamid-6,6-Schlingenware wird nacheinander mit einer Fondflotte foulardiert und mit zwei Reservierungspasten bespritzt. Die Fondflotte enthält auf 1000 Gewichtsteile 7 Teile C.I. Acid Brown 298, 1 Teil Natriumacetat, 2 Teile eines Antifrostingmittels und 2 Teile eines alkoholhaltigen Entschäumers; der pH-Wert wird mit Essigsäure auf 5,0 eingestellt. Die erste Reservierungspaste ist farbstofffrei und enthält auf 1000 Gewichtsteile 10 Teile eines benzylierten Dialkylaminethoxylates der Formel II, 15 Teile eines handelsüblichen Verdickers und 1 Teil Ammonsulfat; der pH-Wert wird auf 5,5 eingestellt. Die zweite Reservierungspaste enthält zusätzlich zu den Bestandteilen der ersten noch 1 Teil C.I. Acid Yellow 151. Die vorgereinigte Teppichware wird mit der Fondflotte foulardiert. Anschließend werden die beiden Reservierungspasten aufgespritzt. Nach Dämpfen und Spülen erhält man einen braunen Teppich mit weißer und gelber Punktmusterung.

### Herstellung der Dialkylmonoaminalkoxylat-Verbindungen

### Beispiel 4

1940 g hydriertes Ditalgfettamin (Kettenlänge C₁₆ - C₁₈, Molekulargewicht ca. 470) werden auf 185 °C aufgeheizt und mit 200 g Ethylenoxid portionsweise so versetzt, daß der Druck nach jeder Zugabe 5 bar beträgt. Man läßt ca. 7-8 Stunden reagieren. Für die zweite Reaktionsstufe wird nach Abkühlen ein Katalysator, z. B. KOH, eingetragen und bei Temperaturen von 150-180 °C und Drucken von 2 - 3 bar mit Ethylenoxid und/oder Propylenoxid bis zum gewünschten Alkoxylierungsgrad weiter umgesetzt.

### Beispiel 5

3000 g hydriertes Ditalgfettamin (Kettenlänge C₁₆ - C₁₈, Molekulargewicht ca. 500) werden auf 185 °C aufgeheizt und innerhalb von 8 Stunden portionsweise so mit 400 g Ethylenoxid versetzt, daß der Druck stets 5 bar beträgt. Die zweite Reaktionsstufe wird wie in Beispiel 4 durchgeführt.

### Beispiel 6

1500 g hydriertes Ditalgfettamin (Kettenlänge C₁₆ - C₁₈, Molekulargewicht ca. 500) werden auf 185 °C aufgeheizt. Man versetzt dann mit 200 g Ethylenoxid und Stickstoff, wobei der Gesamtdruck auf 5 bar gehalten wird, der Stickstoff-Partialdruck jedoch 3 bar beträgt. Man läßt ca. 8 Stunden unter diesen Bedingungen reagieren. Die zweite Reaktionsstufe wird wie in Beispiel 4 durchgeführt.

### Beispiel 7

800 g hydriertes Dikokosfettamin (Kettenlänge C₁₂ - C₁₄, Molekulargewicht ca. 395) werden auf 170 °C aufgeheizt und innerhalb von 4 Stunden portionsweise so mit 100 g Ethylenoxid versetzt, daß der Druck stets auf 4 bar gehalten wird. Anschließend wird die zweite Reaktionsstufe wie in Beispiel 4 vorgenommen.

### Quaternierung der Dialkylmonoaminalkoxylat-Verbindung

### Beispiel 8

38,5 kg hydriertes Ditalgaminethoxylat (Kettenlänge C₁₆ - C₁₈ mit 31 Mol angelagertem Ethylenoxid) werden mit 20 kg Wasser und 5 kg Diethylenglykolmonobutylether auf 80 °C erwärmt. Dann wird mit 2,6 kg Benzylchlorid versetzt und 5 Stunden bei 80 °C unter Rühren quaterniert.

## Patentansprüche

1. Verwendung von Dialkylmonoaminalkoxylat-Verbindungen der allgemeinen Formel 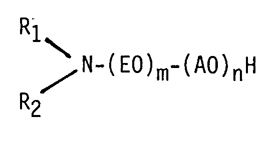 worin
R₁ und R₂ Fettalkylreste mit C₈-C₂₂, gleich oder verschieden, gesättigt oder ungesättigt,
E Ethylen-Rest,
A Ethylen- oder Propylen-Rest ,
m 1 bis 2,
n 1 bis 50
bedeuten, in mindestens teilweise quaternierter Form in farbstofffreien oder farbstoffhaltigen Reservierungspasten zum Herstellen von Farbmusterungen auf synthetischem Polyamidfasermaterial unter oder auf Färbungen mit anionischen Farbstoffen, wobei die Reservierung im pH-Bereich zwischen 3 und 11 unabhängig vom herrschenden pH des Systems wirksam ist.

2. Verwendung der Verbindungen nach Anspruch 1, dadurch gekennzeichnet, daß sie in einer Menge von 1 bis 100 Gewichtsteilen, vorzugsweise 2 bis 30 Gewichtsteilen, bezogen auf 1000 Gewichtsteile Reservierungspaste, eingesetzt werden.

## Claims

1. Use of alkoxylated dialkylmonoamine compounds with the general formula 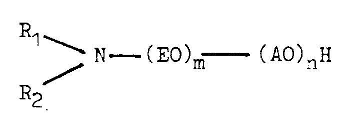 wherein denote
R₁ and R₂ fatty alkyl radicals having 8 to 22 carbon atoms, identical or different, saturated or unsaturated,
E ethylene radical,
A ethylene radical or propylene radical,
m 1 to 2,
n 1 to 50,
in at least partially quaternized form in reserve pastes free of or containing dyestuffs, for the production of colour designs on synthetic polyamide-fibrous material under or upon dyeings with anionic dyestuffs, the reserve effect in the pH range between 3 and 11 being independent of the pH value ruling in the system.

2. Use of the compounds according to claim 1, characterized in that they are used in a quantity of 1 to 100, preferably 2 to 30 parts by weight, in relation to 1000 parts by weight of the reserve paste.

## Revendications

1. Emploi de composés dialkylmonoamines alcoxylés de formule générale 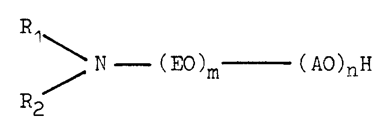 dans laquelle représentent
R₁ et R₂ de radicaux alkyle gras ayant de 8 à 22 atomes de carbone, identiques où différents, saturés ou non-saturés,
E un radical éthylène,
A un radical éthylène ou propylène,
m un nombre de 1 à 2,
n un nombre de 1 à 50,
en forme au moins partiellement quaternisée, dans pâtes de réserve non contenants ou contenants de colorants, pour produire de dessins colorés sur matériel en fibres polyamide synthetique sous ou sur teintures avec de colorants anioniques, l'effet de réserve dans la domaine des pH entre 3 et 11 étant indépendant du régime pH régnant dans le systéme.

2. Emploi des composés selon revendication 1, caracterisé en ce qu'on emploie une quantité de 1 à 100, de préférence de 2 à 30, parts en poids relativement à 1000 parts en poids de pâte de réserve.
